(19) 

Europäisches Patentamt
European Patent Office
Office européen des brevets

(11) **EP 4 481 055 A1**

(12) **EUROPEAN PATENT APPLICATION**

(43) Date of publication:
**25.12.2024 Bulletin 2024/52**

(51) International Patent Classification (IPC):
*C12Q 1/6809* (2018.01)    *G16B 20/20* (2019.01)

(21) Application number: **23181093.8**

(22) Date of filing: **22.06.2023**

(52) Cooperative Patent Classification (CPC):
(C-Sets available)
**C12Q 1/6809; G16B 20/20**                (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Beiersdorf AG**
**22529 Hamburg (DE)**

(72) Inventors:
• **BIENKOWSKA, Agata**
**20535 Hamburg (DE)**

• **GRÖNNIGER, Elke**
**20255 Hamburg (DE)**
• **FALCKENHAYN, Cassandra**
**20249 Hamburg (DE)**
• **KRISTOF, Boris**
**25469 Halstenbek (DE)**
• **SÖHLE, Jörn**
**28355 Bremen (DE)**

(74) Representative: **Haseltine Lake Kempner LLP**
**Bürkleinstrasse 10**
**80538 München (DE)**

(54) **FACIAL AGING PROGRESSION DETERMINATION OF A HUMAN INDIVIDUAL**

(57)    The present invention relates to a method for determining the predicted facial aging progression of human skin comprising providing human skin cells, determining a methylation level of at least one CpG-dinucleotides, or the expression level of at least one gene and determining the predicted facial aging progression of said skin cells by comparing said determined methylation level with empirically determined data representing a correlation between the methylation level of the CpG-nucleotide and the observed facial aging progression of at least one human individual, or determining the predicted facial aging progression of said skin cells by comparing said determined gene expression level with empirically determined data representing a correlation between the gene expression level and the observed facial aging progression of at least one human individual.

Fig. 1

EP 4 481 055 A1

(52) Cooperative Patent Classification (CPC): (Cont.)

C-Sets
**C12Q 1/6809, C12Q 2537/164, C12Q 2537/165,
C12Q 2545/113, C12Q 2600/118, C12Q 2600/154,
C12Q 2523/125**

**Description**

FIELD OF THE INVENTION

The present invention relates to the determination of the predicted facial aging progression of human skin by analysing the epigenetic pattern of particular sites of the DNA or by analysing the expression level of particular genes. The methylation levels at CpG sites or gene expression levels of a skin sample are evaluated to determine the predicted facial aging progression of human skin accurately. The predicted facial aging progression is then correlated to the observed facial aging progression with a mean absolute error of less than 12 years.

BACKGROUND OF THE INVENTION

Aging is a time-dependent general loss of functionality and fitness across the lifespan of an individual. The accumulation of molecular damage is considered one of the key hallmarks of aging. For example, epigenetic changes of DNA are known to occur over time and have been recognised as indicators of the extent of the aging process in human individuals. In particular, the extent of methylation of CpG dinucleotides has been identified as being useful in methods of determining biological age and predicting chronological age. Such known methods are based on the analysis of various CpG-dinucleotides in both skin cells and blood cells. A good correlation between the chronological age and the biological age of an individual was achieved with methods that evaluate the extent of methylation of CpG dinucleotides. In general terms, biological age relates to the overall health status of an individual. Methods of predicting biological age can be useful in addressing a host of questions in development biology, cancer and aging research as discussed in, for example, Horvath, Genome Biology 2013, 14:R115.

Despite the development of such methods, the complexity and heterogeneity of the aging process present significant challenges in unravelling its intricacies. The lack of reliable biomarkers poses a challenge in understanding the aging process, thus developing interventions to promote healthy aging. Almost all known methods of predicting aging focus on estimating chronological age, as described above.

There is growing interest to understand the factors contributing to aging, wherein such models can involve the use of skin tissue. Measuring and monitoring characteristics of the skin on a molecular level could be useful in many applications, such as identifying active agents to minimise the visual signs of aging.

It is therefore desirable to provide a method to determine aging of the skin of an individual.

SUMMARY OF THE INVENTION

The present invention is defined in the appended claims.

In accordance with a first aspect, there is provided a method for determining the predicted facial aging progression of human skin comprising:

a) providing human skin cells;
b) determining a methylation level of at least one CpG-dinucleotide of a specific region of at least one chromosome of said skin cells or detecting expression level of at least one gene of said skin cells; and
c) determining the predicted facial aging progression of said skin cells by comparing said determined methylation level with empirically determined data representing a correlation between the methylation level of said CpG-nucleotide and the observed facial aging progression of at least one human individual or determining the predicted facial aging progression of said skin cells by comparing said determined gene expression level with empirically determined data representing a correlation between the gene expression level and the observed facial aging progression of at least one human individual.

In accordance with a second aspect, there is provided a method of testing an active agent comprising the method according to the first aspect, further comprising the steps of:

d) contacting the skin cells of step a) with an active agent;
e) determining the predicted facial aging progression of the skin cells of step d) according to the method of the first aspect; and
f) comparing the predicted facial aging progression determined in steps a) to c) with the predicted facial aging progression determined in step e).

In accordance with a third aspect, there is provided a composition comprising one or more active agents identified by the method of the second aspect.

In accordance with a fourth aspect, there is provided the use of DNA methylation level or gene expression level for determining the predicted facial aging progression of human skin according to the method of the first aspect.

In accordance with a fifth aspect, there is provided a computer-readable medium having stored computer-executable instructions for causing a computer to perform a method for determining the predicted facial aging progression of human skin comprising:

a) inputting at least one value of a determined methylation level of at least one CpG-dinucleotide of a specific region of at least one chromosome of said skin cells or inputting at least one value of a determined expression level of at least one gene of said skin cells;

b) comparing said value of the determined methylation level with stored data representing a correlation between the methylation level of said CpG-dinucleotide and the observed facial aging progression of at least one human individual or comparing said value of gene expression level with stored data representing a correlation between the gene expression level and the observed facial aging progression of at least one human individual; and

c) displaying the predicted facial aging progression.

In accordance with a sixth aspect, there is provided a kit for determining the predicted facial aging progression of human skin according to the method of the first aspect, comprising at least one oligonucleotide primer for amplifying and/or sequencing at least one CpG-nucleotides of at least one nucleotide sequence or at least one oligonucleotide primer for amplifying and/or sequencing fractions of at least one exon of at least one nucleotide sequence.

Certain embodiments of the present invention may provide one or more of the following advantages:

- desired ease of sample collection from the individual;
- desired ease of obtaining bioinformatic data;
- desired cost-effective data collection;
- desired accuracy of facial aging progression prediction;
- desired ease of identifying active agent to control facial aging progression; and
- desired ease of predicting the effect of active agent on the facial aging progression over time.

The details, examples and preferences provided in relation to any particular one or more of the stated aspects of the present invention apply equally to all aspects of the present invention. Any combination of the embodiments, examples and preferences described herein in all possible variations thereof is encompassed by the present invention unless otherwise indicated herein, or otherwise clearly contradicted by context.

BRIEF DESCRIPTION OF THE DRAWINGS

The invention will further be illustrated by reference to the following figures:

Fig. 1: graph represents a correlation of predicted facial aging progression with observed facial aging progression based on the test set using the DNA methylation method; Pearson correlation ($R^2$), mean absolute error (MAE), and significance of correlation (p) shown;

Fig. 2: graph represents a correlation of predicted facial aging progression with observed facial aging progression based on the independent data set; using the DNA methylation method; Pearson correlation ($R^2$), mean absolute error (MAE), and significance of correlation (p) shown;

Fig. 3: graph represents a correlation of predicted facial aging progression with observed facial aging progression based on the test set using the DNA methylation method based on LMRs; Pearson correlation ($R^2$), mean absolute error (MAE), and significance of correlation (p) shown;

Fig. 4: graph represents a correlation of predicted facial aging progression with observed facial aging progression based on the independent data set; using the DNA methylation method based on LMRs; Pearson correlation ($R^2$), mean absolute error (MAE), and significance of correlation (p) shown;

Fig. 5: graph represents a correlation of predicted facial aging progression with observed facial aging progression based on the test set using the gene expression method; Pearson correlation ($R^2$), mean absolute error (MAE), and significance of correlation (p) shown;

It is understood that the following description and references to the figures concern exemplary embodiments of the present invention and shall not be limiting the scope of the claims.

DETAILED DESCRIPTION

The present invention is based on the finding that the methylation level of CpG nucleotides or gene expression level can be used to predict the facial aging progression of human skin with good accuracy.

The signs of aging can be seen on the skin in a number of ways, such as skin texture, tone and laxity. A key area of the body where the signs of aging are immediately noticeable is the face. The overall appearance of the face can be evaluated to determine the facial age, which is typically carried out by visual analysis, which is referred to herein as "visual facial age". There are various methods that can be implemented to determine the visual facial age. Visual facial age is a reliable measure of aging as it encompasses the cumulative effects of several factors, such as sun exposure, lifestyle, and genetic predisposition, that contribute to the aging process. Therefore, a number of features are taken into consideration, rather than focussing on a single feature, which may provide a better overall picture of the extent of aging. Therefore, facial age offers a more holistic perspective on aging speed, as it considers various features of the face, including skin texture, tone, and laxity, which are influenced by multiple biological processes.

In the present disclosure the visual facial age is determined using a method which involves the visual analysis of the face of an individual by trained individuals, also known herein as "judges". In this method, judges are asked to evaluate the visual appearance of aging signs in an individual's face and award a facial age to the individual. The facial age awarded to an individual is based on the overall appearance of the skin on the face, specifically the extent of wrinkles like crow's feet, aging spots and skin tone. The facial age associated with an individual can be between 1 and 120 years and the assessment of the facial age has a subjective element, as it relies on empirical values. Therefore, multiple judges are asked to provide their assessment of a single individual and the facial age is an average of the assessments provided by the judges. In this method, the assessment of at least 10 judges is applied, for example, at least 12 judges, at least 15 judges, at least 18 judges, at least 20 judges, at least 25 judges, at least 30 judges.

The visual facial age was also compared with the chronological age of an individual. The difference between the visual facial age and the chronological age was determined and is referred to herein as "observed facial aging progression". The observed facial aging progression provides an indication of the extent of aging over time and its correlation with the chronological age. For example, if:

- the visual facial age is 35 years and the chronological age is 35 years, the observed facial aging progression will be 0 years. This means that the facial aging has progressed in line with the chronological age;
- the visual facial age is 40 years and the chronological age is 35 years, the observed facial aging progression is 5 years. This means that the facial aging has progressed faster than the chronological age; and
- the visual facial age is 30 years and the chronological age is 35 years, the observed facial aging progression is -5 years. This means that the facial aging has progressed slower than the chronological age.

As used herein, the term "predicted facial aging progression" refers to the difference between the visual facial age and the chronological age of an individual and is based on the epigenetic level of DNA and/or gene expression level. The epigenetic level of DNA and/or gene expression level is determined in skin samples. The predicted facial aging progression as disclosed herein is determined using the method of the first aspect of this invention. In particular, the methylation level of specific CpG-dinucleotides or expression level of specific genes are assessed as part of this invention.

The DNA methylation level can be determined using a DNA methylation level detection method. Examples of DNA methylation level detection methods include, but are not limited to: (a) base-resolution and sequencing approaches like Whole Genome Bisulfite Sequencing (WGBS aka MethylC-Seq), Enzymatic Methyl-Seq (EM-Seq), Anchor-Based Bisulfite Sequencing (ABBS), Post-Bisulfite Adaptor Tagging (PBAT), Reduced Representation Bisulfite Sequencing (RRBS) or Shotgun Bisulfite Sequencing (BS-Seq), (b) base-resolution and microarray approaches either based on oligonucleotides (e.g., Reinders et al. "Genome-wide, high-resolution DNA methylation profiling using bisulfite-mediated cytosine conversion"; Genome Res. 2008 Mar;18(3):469-76) or bead-chips (e.g., Illumina), (c) non-base resolution and sequencing approaches like Methylated DNA Immunoprecipitation-Sequencing (MeDIP-Seq), Methylated-CpG Island Recovery Assay (MIRA), Methylation-Sensitive Restriction Enzyme Sequencing (MRE-Seq) or MethylCap-Seq (aka MBDCap-Seq), (d) non-base resolution and microarray approaches like MeDIP-Chip or Comprehensive High-throughput Arrays for Relative Methylation (CHARM) and (e) targeted approaches like targeted bisulfite sequencing, targeted enzymatic methyl-seq, ligation-mediated PCR, Methylation-Specific PCR (MS-PCR), Methylation-Sensitive High Resolution Melting (MS-HRM) or Combined Bisulfite Restriction Analysis (COBRA).

The DNA methylation level can be represented either as beta- or m-values. Beta-values are defined as number of detected methylated (M) molecules divided by the sum of methylated (M) and unmethylated (U) molecules. In other words, M equals number of detected methylated molecules or signal intensities of methylated probes; U equals number of detected unmethylated molecules or signal intensities of unmethylated probes: (see equation 1).

$$beta\ value = \frac{M}{M + U} \qquad \text{(Equation 1)}$$

M-values are defined as the log2 ratio of the number of methylated versus unmethylated molecules (see equation 2). Complementary, number of detected methylated and unmethylated molecules can also represented by the signal intensities of methylated and unmethylated probes, respectively.

$$m\ value = log2\left(\frac{M}{U}\right) \qquad \text{(Equation 2)}$$

In addition to the DNA methylation levels determined in the detection methods disclosed above, in some examples, specific regions known as low methylated regions (LMRs) are used to carry out the methods according to the present invention. LMRs are characterised by relatively low levels of DNA methylation and frequently coincide with gene regulatory regions such as enhancers and promoters. LMRs can be determined in a number of known ways, including the application of, for example, the algorithm MethylSeekR.

The gene expression level can be determined using a gene expression level detection method. Examples of gene expression level detection methods include, but are not limited to: (a) sequencing-based high-throughput assays such as Ribonucleic Acid-Sequencing (RNA-Seq), single cell RNA-Sequencing (scRNA-Seq), spatial transcriptomics (e.g., Visium Spatial Gene Expression and FISSEQ), Serial Analysis of Gene Expression (SAGE), or variations of Serial Analysis of Gene Expression (SAGE) (e.g., LongSAGE, RL-SAGE and SuperSAGE), (b) hybridization-based high- to medium-throughput assays such as microarrays (e.g., in situ synthesized oligonucleotide-based, cDNA-based, bead-based and tiling-based microarray platforms), Luminex xMAP Technology, NanoString nCounter technology, Multiplexed Error Robust Fluorescence In Situ Hybridization (MERFISH), digital spatial transcriptomics (e.g., GeoMx DSP) or RNAscope ISH, (c) polymerase chain reaction (PCR) based medium- to low-throughput assays such as digital PCR or quantitative and reverse transcription PCR (qRT-PCR and RT-PCR) e.g., TaqMan-assay and Low Density Arrays (LDA) and (d) hybridization-based medium- to low-throughput assays such as QuantiGene RNA Assays, Fluorescent in situ hybridization (FISH), Northern blotting, Dot-Blot or Slot-Blot.

The gene expression level may be represented based on counts (e.g., raw counts, raw counts normalized to housekeeping genes and or internal standards as well as normalized counts like TPMs, RPKMs and FPKMs), fluorescence signal intensities (e.g., of hybridization-based assays) or a change (e.g., log2FC or FC).

The visual facial age, observed facial aging progression and/or predicted facial aging progression may be influenced by many parameters such as genetic background, age, exposure to ultraviolet light, smoking, exposure to pollution and/or repeated facial expressions.

The visual facial age, observed facial aging progression and/or predicted facial aging progression may be influenced by a number of factors such as, for example, protecting the skin from sun, moisturising, refraining from smoking and eating a healthy diet. The facial aging progression may also be affected by the use of active agents.

In certain embodiments the skin cells used according to the method of the invention are harvested from a human individual. In certain embodiments, the human skin cells used according to the method of the invention are obtained by harvesting the entire skin sample required from the individual. In certain embodiments, the human skin cells used according to the method of the invention are obtained by harvesting a skin sample from the arm, face, neck, décolleté and/or hand. In certain embodiments, the human skin cells used according to the method of the invention are obtained by culturing the skin cells using an *in vitro* method.

Harvesting a sample from the individual may be carried out using suction blistering, punch biopsy, shave biopsy or during any surgical procedure such as plastic surgery, lifting, grafting, or the like.

The skin samples may be taken from the epidermis or dermis.

Human skin cells may be cultured from a small sample of skin cells harvested from an individual. The harvested human skin cells are grown in vitro in a vessel such as a petri dish in a medium or substrate that supplies essential nutrient.

The human skin cells used may be a mixture of harvested cells and cultured cells.

In certain embodiments, the specific region of the chromosome comprising CpG-dinucleotides according to the invention may be coding regions or non-coding regions. In certain embodiments, the CpG dinucleotides may be present in a coding region and/or non-coding region. The CpG dinucleotides may be found in a single specific region or in different specific regions.

In certain embodiments, the method of the first aspect further comprises the step of estimating the predicted facial aging progression of human individuals.

The correlation between the predicted facial aging progression and observed facial aging progression is high using the method according to the invention, wherein the difference between the predicted facial aging progression and the observed facial aging progression can be expressed using the mean absolute error (MAE). The mean absolute error (MAE) of the facial aging progression determined by the method described herein, is no more than about no more than about 12 years, no more than about 11 years, no more than about 10 years, no more than about 9 years, no more than about 8 years, no more than about 7 years, no more than about 6 years, no more than about 5 years, no more than about 4 years, no more than about 3 years, no more than about 2 years, no more than about 1 year.

The difference in observed facial aging progression and predicted facial aging progression may be determined for a single individual. The deviation of a single data point from the best fit line superimposed onto the data points from all individuals can vary from about 0 years to about 12 years. For example, the deviation of a single data point from the best fit line would be about 0 years, about 1 year, about 2 years, about 3 years, about 4 years, about 5 years, about 6 years, about 7 years, about 8 years, about 9 years, about 10 years, about 11 years, about 12 years.

In certain embodiments, the predicted facial aging progression of an unknown individual is determined as described above. From this predicted facial aging progression, the observed facial aging progression of the individual could be estimated by applying the expected MAE for the CpG data points used or by applying the expected MAE for the gene expression levels used.

The visual facial age, observed facial aging progression and/or predicted facial aging progression may also be reduced by applying active agents onto the skin cells in the form of pharmaceutical and/or cosmetic agents. By means of the method of the present invention, the mid-term and/or long-term reduction of facial aging upon application of an active agent may also be predicted in a reliable manner. This prediction may be made at an early stage and without the need of monitoring the phenotype progression of the skin over a long period of time, which can be costly and time consuming. Without wishing to be bound by theory, it is considered that the predictive method of facial aging progression with time using the method of the invention is possible due to the connection that the present inventors have discovered between the specific biological changes determined in the method and the associated facial aging progression. Such a method is not known or contemplated in the art. The active agent may contact the skin cells *in vitro* or *in vivo*. Using the *in vitro* method, the active agent is added to the culture medium of skin cells. Using the *in vivo* method, the skin cells of a human individual may be contacted with the active agent using topical, subcutaneous or intradermal administration.

An "active agent" as used herein is any agent that has a therapeutic effect and/or cosmetic effect on the individual. A therapeutic effect is the treatment and/or prevention of a disease. A cosmetic effect is the improvement of appearance, such as treating and/or preventing the signs of molecular aging. The cosmetic effect may be the reduction in the facial aging progression, which includes the observed facial aging progression and/or predicted facial aging progression. In certain embodiments the active agent is a cosmetic agent. A cosmetic agent when applied to an individual may promote attractiveness, alter appearance, beautify and/or cleanse. The cosmetic agent may also prevent and/or treat the signs of phenotypical aging of human skin which are, for example, wrinkle formation, pale complexion, reduced wound healing capacity, loss of elasticity and tightness.

In certain embodiments, the predicted facial aging progression is determined before and after contacting the skin cells with an active agent. For example, the predicted facial aging progression is determined, the skin cells are contacted with an active agent, followed by determining the predicted facial aging progression. The predicted facial aging progression before and after contacting the skin cells with an active agent are compared. A reduction in the predicted facial aging progression after treatment indicates a therapeutic effect and/or cosmetic effect of the active agent. The time between contacting the skin cells with an active agent and determining the predicted facial aging progression may be varied. The steps of contacting the skin cells with an active agent and determining the predicted facial aging progression may be repeated to provide information about the effect of the active agent on the skin cells over time. This method may have the advantage of providing fast and cost-effective way to identify active cosmetic and/or pharmaceutical compounds and/or extracts, especially to treat the phenotype properties of the skin such as the effects of aging. In certain embodiments active agents identified by the method according to the invention are formulated to produce dermatologic compositions. The dermatological composition may be a cosmetic composition and/or a pharmaceutical composition. The dermatological compositions may be used to reduce the predicted facial aging progression and/or observed facial aging progression.

The present disclosure may be described by one or more of the following paragraphs:

A. A method for determining the predicted facial aging progression of human skin comprising:

a) providing human skin cells;
b) determining a methylation level of at least one CpG-dinucleotide of a specific region of at least one chromosome of said skin cells or detecting expression level of at least one gene of said skin cells; and
c) determining the predicted facial aging progression of said skin cells by comparing said determined methylation level with empirically determined data representing a correlation between the methylation level of said CpG-nucleotide and the observed facial aging progression of at least one human individual or determining the predicted facial aging progression of said skin cells by comparing said determined gene expression level with empirically determined data representing a correlation between the gene expression level and the observed facial aging progression of at least one human individual.

B. The method of paragraph A, wherein the skin cells are harvested from a human individual.

C. The method of paragraph A, wherein the skin cells are cultured *in vitro.*

D. The method according to any one of the preceding paragraphs, wherein the methylation level of step b) is determined using a DNA methylation level detection method.

E. The method according to paragraph D, wherein the DNA methylation level detection method targets a low methylated region.

F. The method according to paragraph D, wherein the methylation level determined from the DNA methylation level detection method is represented as beta- or m-values.

G. The method according to paragraph D to paragraph F wherein the DNA methylation level detection method is selected from a) a base-resolution and sequencing approach, b) a base-resolution and microarray approach, c) a non-base resolution and sequencing approach, d) non-base resolution and microarray approach or e) a targeted approach.

H. The method according to paragraph G, where the base-resolution and sequencing approach is selected from Whole Genome Bisulfite Sequencing (WGBS), Enzymatic Methyl-Seq (EM-Seq), Anchor-Based Bisulfite Sequencing (ABBS), Post-Bisulfite Adaptor Tagging (PBAT), Reduced Representation Bisulfite Sequencing (RRBS) or Shotgun Bisulfite Sequencing (BS-Seq).

I. The method according to paragraph G, wherein the base-resolution and microarray approach is based on oligonucleotides or bead-chips.

J. The method according to paragraph G, wherein the non-base resolution and sequencing approach is selected from Methylated DNA Immunoprecipitation-Sequencing (MeDIP-Seq), Methylated-CpG Island Recovery Assay (MIRA), Methylation-Sensitive Restriction Enzyme Sequencing (MRE-Seq) or MethylCap-Seq (MBDCap-Seq).

K. The method according to paragraph G, wherein the non-base resolution and microarray approach is selected from MeDIP-Chip or Comprehensive High-throughput Arrays for Relative Methylation (CHARM)

L. The method according to paragraph G, wherein the targeted approach is selected from targeted bisulfite sequencing, targeted enzymatic methyl-seq, ligation-mediated PCR, Methylation-Specific PCR (MS-PCR), Methylation-Sensitive High Resolution Melting (MS-HRM) and Combined Bisulfite Restriction Analysis (COBRA).

M. The method according to any one of paragraphs A to C, wherein the expression level of step b) is determined using a gene expression level detection method.

N. The method according to any one of paragraph M, wherein the gene expression level detection method is selected from a) sequencing-based high-throughput assays, b) hybridization-based high- to medium-throughput assays, c) polymerase chain reaction (PCR) based medium- to low-throughput assays or d) hybridization-based medium- to low-throughput assays

O. The method according to paragraph N, wherein the sequencing-based high-throughput assay is selected from Ribonucleic Acid-Sequencing (RNA-Seq), single cell RNA-Sequencing (scRNA-Seq), spatial transcriptomics, Serial Analysis of Gene Expression (SAGE) and variations of Serial Analysis of Gene Expression (SAGE).

P. The method according to paragraph O, wherein the spatial transcriptomics are selected from Visium Spatial Gene Expression and FISSEQ.

Q. The method of paragraph O wherein the variations of Serial Analysis of Gene Expression (SAGE) are selected from LongSAGE, RL-SAGE and SuperSAGE.

R. The method according to paragraph N, wherein the hybridization-based high- to medium-throughput assay is selected from microarrays, Luminex xMAP Technology, NanoString nCounter technology, Multiplexed Error Robust Fluorescence In Situ Hybridization (MERFISH), digital spatial transcriptomics and RNAscope ISH.

S. The method according to paragraph R, wherein the microarrays are selected from in situ synthesized oligonucleotide-based, cDNA-based, bead-based and tiling-based microarray platforms.

T. The method according to paragraph R, wherein the digital spatial transcriptomics is GeoMx DSP.

U. The method according to paragraph N, wherein the polymerase chain reaction (PCR) based medium- to low-throughput assay is selected from digital PCR, quantative PCT, reverse transcription PCR and Low-Density Arrays (LDA).

V. The method according to paragraph U, wherein the reverse transcription PCR includes qRT-PCR and RT-PCR.

W. The method according to paragraph U or paragraph V, wherein the reverse transcription PCR is TaqMan-assay.

X. The method according to paragraph N, wherein the hybridization-based medium-to low-throughput assay is selected from QuantiGene RNA Assays, Fluorescent in situ hybridization (FISH), Northern blotting, Dot-Blot and Slot-Blot.

Y. The method according to paragraphs A, B, or D to X, further comprising the step of estimating the observed facial aging progression of the human individual.

Z. The method of paragraph Y, wherein the predicted facial aging progression is estimated to be within 12 years of the observed facial aging progression.

AA. The method according to any one of the preceding paragraphs further comprising the step of contacting the skin cells with an active agent.

BB. The method according to paragraph AA, wherein the predicted facial aging progression is determined before and after contacting the skin cells with an active agent.

CC. A method of testing an active agent comprising the method of any one of paragraphs A to X, further comprising the steps of:

  d) contacting the skin cells of step a) with an active agent;
  e) determining the predicted facial aging progression of the skin cells of step d) according to the method of any one of paragraphs A to X; and
  f) comparing the predicted facial aging progression determined in steps a) to c) with the predicted facial aging progression determined in step e).

DD. The method of paragraph CC, wherein step d) is carried out either *in vivo* or *in vitro.*

EE. The method of paragraph CC or paragraph DD, wherein the active agent is a cosmetic agent and/or a therapeutic agent.

FF. The method of any one of paragraphs CC to EE to identify an active agent that prevents and/or treats the signs of phenotypical aging of human skin.

GG. A dermatological composition comprising one or more active agents identified by the method of any one of paragraphs CC to FF.

HH. Use of the composition of paragraph GG for reducing the predicted facial aging progression.

II. Use of DNA methylation levels or gene expression levels for determining the predicted facial aging progression of human skin according to the method of any one of paragraphs A to X.

JJ. Computer-readable medium having stored computer-executable instructions for causing a computer to perform a method for determining the predicted facial aging progression of human skin comprising:

  d) inputting at least one value of a determined methylation level of at least one CpG-dinucleotide of a specific region of at least one chromosome of said skin cells or inputting at least one value of a determined expression level of at least one gene of said skin cells;
  e) comparing said value of the determined methylation level with stored data representing a correlation between the methylation level of said CpG-dinucleotide and the observed facial aging progression of at least one human individual or comparing said value of gene expression level with stored data representing a correlation between the gene expression level and the observed facial aging progression of at least one human individual; and
  f) displaying the predicted facial aging progression.

KK. Computer-readable medium according to paragraph JJ, wherein said stored data comprise at least one linear regression equation.

LL. A kit for determining the predicted facial aging progression of human skin according to the method of any one of the paragraphs A to X comprising at least one oligonucleotide primer for amplifying and/or sequencing at least one CpG-nucleotides of at least one nucleotide sequence or at least one oligonucleotide primer for amplifying and/or sequencing fractions of at least one exon of at least one nucleotide sequence.

In certain embodiments, the method according to the invention may have one or more of the following effects:

- efficient method of predicting the facial aging progression;
- reliable correlation between observed facial aging progression and predicted facial aging progression;
- reduced time required to determine the predicted facial aging progression;
- efficient method to determining the effect of pharmaceutical or cosmetic agents on facial aging progression;
- efficient method to predict the effect of active agent on facial aging progression;
- effective method to predict the reduction in facial aging progression over time as a result of applying active agents, e.g. applying active agent over long period of time such as one year or more; and
- efficient method to predict the effect of active agents over extended periods of time for an individual, providing a personalised prediction.

It should be noted that the present invention may comprise any combination of the features and/or limitations referred to herein, except for combinations of such features which are mutually exclusive. The foregoing description is directed to particular embodiments of the present invention for the purpose of illustrating it. It will be apparent, however, to one skilled in the art, that many modifications and variations to the embodiments described herein are possible. All such modifications and variations are intended to be within the scope of the present invention, as defined in the appended claims.

EXAMPLES

Example 1:

Suction blisters of 7mm diameter were collected from the volar forearms of 461 female participants of the population-based Study of Health in Pomerania (SHIP-TREND-1) (Volzke et al., "Cohort Profile Update: The Study of Health in Pomerania (SHIP)"; Int J Epidemiol, 51, e372-e383, 2022) aged between 29 and 84 years. Suction blisters were obtained by applying a negative pressure of 180 mbar for 30 min followed by 320 mbar until blister formation. The roof of the suction blisters was prepared with surgery scissors and tweezers, and after snap freezing in liquid nitrogen stored at -80 °C until further use.

The DNA from the suction blister roofs of each of the 461 participants was isolated using the QIAmp DNA Investigator Kit (Qiagen) following the manufacturer's instructions. DNA methylation patterns of the 461 female epidermal samples were determined via Infinium® MethylationEPIC BeadChip arrays (Illumina).

Methylation data analysis was carried out using the R Bioconductor package Minfi (Aryee et al., "Minfi: a flexible and comprehensive Bioconductor package for the analysis of Infinium DNA methylation microarrays"; Bioinformatics, 30, 1363-9, 2014) and limma (Ritchie et al., "limma powers differential expression analyses for RNA-sequencing and microarray studies"; Nucleic Acids Res, 43, e47, 2015). Specifically, raw .idat files were read and preprocessed. Methylation loci (probes) were filtered for high detection p-value (P>0.01, as provided by Minfi), location on sex chromosomes, ability to self-hybridize, and potential SNP contamination. Array normalization was performed using the *preprocessQuantile* function, available in Minfi. Quality control was performed after every preprocessing step.

In addition to the DNA methylation level, portrait pictures were taken from each of the 461 study participants. Each volunteer had to close their eyes, cover their hair, remove the make-up and jewellery before photographing. The images were taken under following standardized setup: flash diffuser to ensure evenly lit, non-polarized and colour controlled. Based on those pictures, an expert panel consisting of more than 30 experts judged the facial age within a range of 1 to 120 years for each study participant. To avoid biases in judgment of the volunteers, the pictures were randomly presented to each expert in such a way that none of the 30 experts had judged the volunteers in the same order. Finally, the average of those expert judgments resulted in a volunteer's individual facial age.

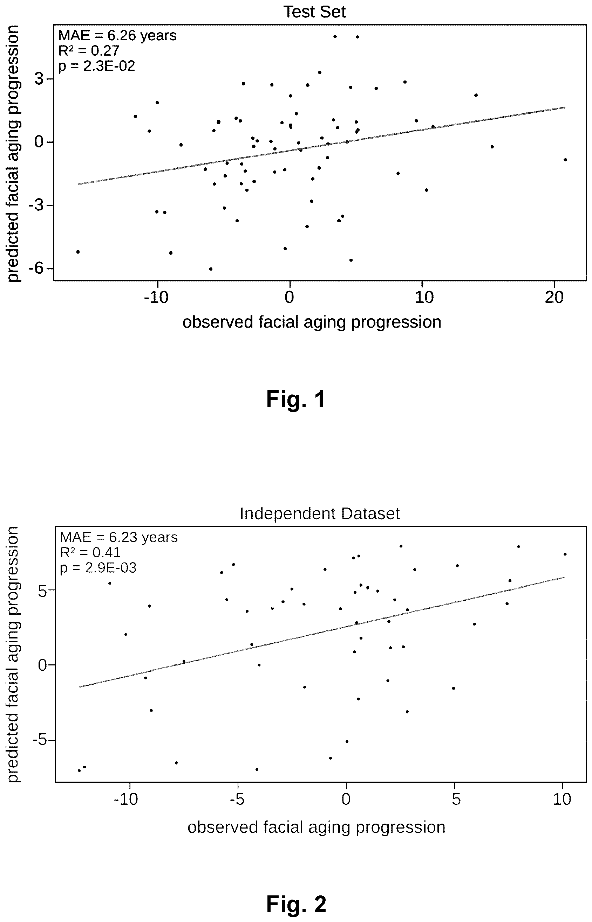

A sample group of 378 was then split in two, with 302 epidermal samples of female participants used for training and 76 epidermal samples of female participants used for testing. The difference between the visual facial age and the chronological age was calculated by subtracting the chronological age from the visual facial age to obtain the observed facial aging progression. The training of the facial aging progression predictor was conducted with the beta-values of the training dataset applying the *cv.glmnet* function (alpha = 0, lambda = 295.39) of the *glmnet* R package (Friedman et al., "Regularization Paths for Generalized Linear Models via Coordinate Descent"; Journal of Statistical Software, 33, 1-22; 2010) in a 10-fold cross-validation mode using the observed facial aging progression as outcome variable. This method established the empirically determined data as a representation of the correlation between the methylation level of said CpG-nucleotide and the observed facial aging progression of at least one human individual and is also referred to herein as the "trained facial aging progression predictor". The trained facial aging progression predictor was validated with the test set and an independent publicly available dataset (Holzscheck et al., "Multi-omics network analysis reveals distinct stages in the human aging progression in epidermal tissue"; AGING, 12; 2020). Prediction of the facial aging progression for each of the volunteers from the test set of the empirically determined data compared to the corresponding observed facial aging progression resulted in a very high correlation ($R^2 = 0.27$ and $P = 2.3 \times 10^{-2}$, Pearson correlation) and an average absolute prediction error of less than 6.26 years (see Figure 1). Similar holds true for the validation of the trained facial age predictor with an independent dataset ($R^2 = 0.41$ and $P = 2.9 \times 10^{-3}$) with an absolute prediction error of less than 6.23 years (see Figure 2).

Example 2:

Example 1 was then repeated by reducing the determined DNA methylation patterns of the 378 female epidermal samples via Infinium® MethylationEPIC BeadChip arrays (Illumina) down to the DNA patterns of specific low methylated regions (LMRs) which were used as input for the model training.

The LMRs used were determined by combining whole-genome bisulfite sequencing (WGBS) methylation data from epidermis of young and old subjects (Raddatz, G. et al., "Aging is associated with highly defined epigenetic changes in the human epidermis"; EPIGENETICS CHROMATIN; 6(1), 36; 2013) leading to an average strand-specific CpG coverage of 14x. MethylSeekR (Burger, L. et al., "Identification of active regulatory regions from DNA methylation data", NUCLEIC ACIDS RES; 41(16), e155; 2013) was then applied using standard parameters to this dataset to identify skin-specific LMRs and those, which were overlapping with at least one probe of the EPIC Methylation array, were finally selected for further analysis. This led to a set of 40,140 LMRs. For the training of the facial aging progression predictor, the average beta value for each LMR was calculated by considering the CpGs located within that specific region and used as data input following a methodology similar to the one described in Example 1 with parameters set to 0 and lambda set to 19.45.

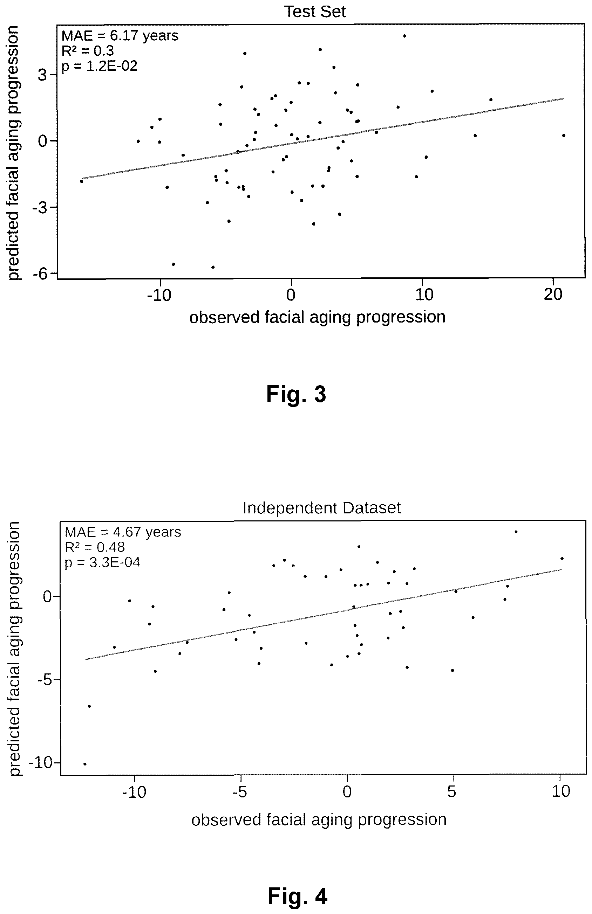

Prediction of the facial aging progression for each of the volunteers from the test set of the empirically determined data compared to the corresponding observed facial aging progression resulted in a very high correlation ($R^2 = 0.3$ and $P = 1.2 \times 10^{-2}$, Pearson correlation) and an average absolute prediction error of less than 6.17 years (see Figure 3). Similar holds true for the validation of the trained facial age predictor with an independent dataset ($R^2 = 0.48$ and $P = 3.3 \times 10^{-4}$) with an absolute prediction error of less than 4.67 years (see Figure 4).

Example 3:

Suction blister roofs of 437 female participants of the population-based Study of Health in Pomerania (SHIP-TREND-1) (Volzke et al., "Cohort Profile Update: The Study of Health in Pomerania (SHIP)"; Int J Epidemiol, 51, e372-e383, 2022) aged between 29 and 84 years were suspended in lysis buffer for RNA extraction and homogenized using an MM 301 bead mill (Retsch). Using the RNeasy Fibrous Tissue Mini Kit (Qiagen) RNA was extracted following the manufacturer's instructions.

Using the TruSeq Library Prep Kit (Illumina) the transcriptome libraries were prepared following the manufacturer's instruction. Sequencing was performed on Illumina's HiSeq system in single end mode with a read length of 50 bp to a final sequencing depth of 100 million reads per sample. Generated raw reads were processed as follows: (i) quality control using Fastqc 0.11.7 (Andrews, S. s-andrews/FastQC. GitHub https://github.com/s-andrews/FastQC), (ii) trimming of read sequences via Trimmomatic 0.36 (Bolger, A. M., Lohse, M. & Usadel, B. Trimmomatic: a flexible trimmer for Illumina sequence data. Bioinformatics 30, 2114-2120 (2014).) with parameters for adapter trimming using the TruSeq adapter sequences, seed mismatches set to 2, palindrome clip threshold set to 30 and simple clip threshold set to 10, parameters for sliding window with a window size set to 4 and the required quality to 20, parameters for leading and trailing as minimum quality required to keep a base was set to 20 and a minimum required length of a read to be kept was set to 40 bp, (iii) mapping of reads against the GRCh38 build of the human transcriptome by Salmon 0.8.1 (Patro, R., Duggal, G., Love, M. I., Irizarry, R. A. & Kingsford, C. Salmon provides fast and bias-aware quantification of transcript expression. Nat. Methods 14, 417-419 (2017).) with parameters "bootstrap" set to 0, "flsd" set to 10 and "flmean" set to 180 and (iv) quantification of reads as transcripts per million (TPM).

Of all 38,892 detected genes those which were not expressed (TPM = 0) in at least one of the volunteers (average TPM = 0) were defined as unexpressed in skin and thus removed from the data table to minimize set of input variables for the final model. Furthermore, genes which could not be annotated to an official human gene symbol were removed as well to ensure cross-reference among different gene expression detection methods and annotation databases. Then, the gene expression data were log10 transformed by applying following formula *transformed gene expression data = log*10(*TPM +* 1). To further reduce input complexity for the model, the coefficient of variation was calculated for each gene as follows

$$CV_i = \frac{standard\ deviation(gene_i)}{mean(gene_i)}$$

and used as cutoff to remove genes with a CV below 0.075 resulting in a final set of 23,539 features. Finally, the sample group was split into 75% training and 25% test set controlling for age and facial age to ensure a similar distribution of facial age among the two generated data sets.

The facial aging progression predictor based on gene expression data was obtained by training a fully connected neural network which was implemented in R (R Core Team. R: A Language and Environment for Statistical Computing. The R Foundation (2018).) using keras (Chollet, F. keras-team/keras. GitHub https://github.com/keras-team/keras.) with a tensorflow backend (Abadi, M. et al. TensorFlow: A system for large-scale machine learning. In 12th USENIX Symposium on Operating Systems Design and Implementation (OSDI 16) 265-283 (The Advanced Computing Systems Association, 2016).). The neural network had the following architecture: (i) input layer with nodes equal to the number of features, (ii) hidden layer 1 with nodes equal to the sqrt of the first layer, (iii) hidden layer 2 with nodes equal to the sqrt of the second layer, and (iv) output layer with one node for the observed facial aging progression. The weights in the two hidden layers were initialized using the He-function (He, K., Zhang, X., Ren, S. & Sun, J. Delving Deep into Rectifiers: Surpassing HumanLevel Performance on ImageNet Classification. Preprint at arXiv:1502.01852 [cs] (2015).) and the "elu" (exponential linear units) activation function (Clevert, D. -A., Unterthiner, T. & Hochreiter, S. Fast and Accurate Deep Network Learning by Exponential Linear Units (ELUs). Preprint at arXiv:1511.07289 [cs] (2015).) was used in all hidden layers. The loss function for the training was defined as mean squared error. To improve the generalization ability of the model the weight decay was implemented, and the regularization factor (L2) was set to 0.1. Furthermore, the model was trained using the stochastic gradient descent with Adam (Kingma, D. P. & Ba, J. Adam: a method for stochastic optimization. Preprint at arXiv:1412.6980 [cs] (2017).) and a learning rate of 0.001 with a batch size of 15 samples for training until 100 epochs. All parameters of the neural network are summarized in Table 1.

**Table 1:** Parameters of the trained neural network to predict the facial aging progression based on gene expression data as input.

| Parameter | Value |
| --- | --- |
| Number of input qenes | 23,539 |
| Number of hidden layers | 2 |
| Activation function | elu |
| Weiqht initialization | He |
| L2-regularization (weight decay) | 0.1 |
| Loss calculation | Mean squared error |
| Optimizer | Adam |
| Learning rate | 0.001 |
| Batch size | 15 |
| Training epochs | 100 |

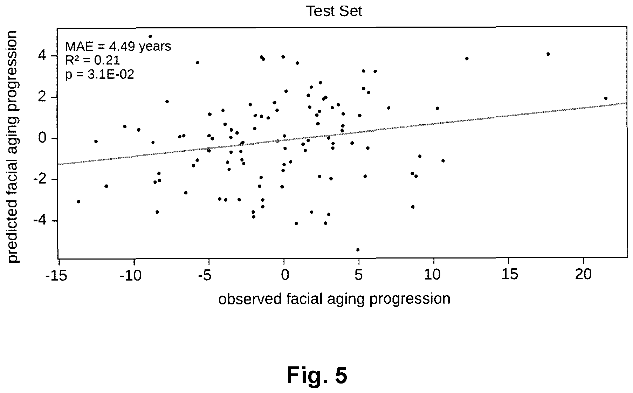

The trained facial aging progression predictor was validated with the test set. Prediction of the facial aging progression for each of the volunteers from the test set of the empirically determined data compared to the corresponding observed facial aging progression resulted in a very high correlation ($R^2 = 0.21$ and $P = 3.1 \times 10^{-2}$, Pearson correlation) and an average absolute prediction error of less than 4.49 years (see Figure 5).

**Claims**

1. A method for determining the predicted facial aging progression of human skin comprising:

    g) providing human skin cells;
    h) determining a methylation level of at least one CpG-dinucleotide of a specific region of at least one chromosome of said skin cells or detecting expression level of at least one gene of said skin cells; and
    i) determining the predicted facial aging progression of said skin cells by comparing said determined methylation level with empirically determined data representing a correlation between the methylation level of said CpG-nucleotide and the observed facial aging progression of at least one human individual or determining the predicted facial aging progression of said skin cells by comparing said determined gene expression level with empirically determined data representing a correlation between the gene expression level and the observed facial aging progression of at least one human individual.

2. The method of claim 1, wherein the skin cells are harvested from a human individual.

3. The method of claim 1, wherein the skin cells are cultured *in vitro*.

4. The method according to any one of the preceding claims, wherein the methylation level of step b) is determined using a DNA methylation level detection method.

5. The method according to claim 4, wherein the DNA methylation level detection method targets a low methylated region.

6. The method according to claim 4 or claim 5, wherein the DNA methylation level detection method is selected from a) a base-resolution and sequencing approach, b) a base-resolution and microarray approach, c) a non-base resolution and sequencing approach, d) non-base resolution and microarray approach or e) a targeted approach.

7. The method according to any one of claims 1 to 3, wherein the expression level of step b) is determined using a gene expression level detection method.

8. The method according to claim 7, wherein the gene expression level detection method is selected from a) sequencing-based high-throughput assays, b) hybridization-based high- to medium-throughput assays, c) polymerase chain reaction (PCR) based medium- to low-throughput assays or d) hybridization-based medium- to low-throughput assays.

9. The method according to claims 1, 2 or 4 to 8, further comprising the step of estimating the observed facial aging progression of the human individual, optionally wherein the predicted facial aging progression is estimated to be within 12 years of the observed facial aging progression.

10. The method according to any one of the preceding claims further comprising the step of contacting the skin cells with an active agent, optionally wherein the predicted facial aging progression is determined before and after contacting the skin cells with an active agent.

11. A method of testing an active agent comprising the method of any one of claim 1 to 8, further comprising the steps of:

j) contacting the skin cells of step a) with an active agent;
k) determining the predicted facial aging progression of the skin cells of step d) according to the method of any one of claims 1 to 8; and
l) comparing the predicted facial aging progression determined in steps a) to c) with the predicted facial aging progression determined in step e).

12. The method of claim 11, wherein step d) is carried out either *in vivo* or *in vitro* and/or, wherein the active agent is a cosmetic agent and/or a therapeutic agent.

13. The method of claims 11 or claim 12 to identify an active agent that prevents and/or treats the signs of phenotypical aging of human skin.

14. A composition comprising one or more active agents identified by the method of any one of claims 11 to 13.

15. Use of DNA methylation levels or gene expression level for determining the predicted facial aging progression of human skin according to the method of any one of claims 1 to 8.

16. Computer-readable medium having stored computer-executable instructions for causing a computer to perform a method for determining the predicted facial aging progression of human skin comprising:

g) inputting at least one value of a determined methylation level of at least one CpG-dinucleotide of a specific region of at least one chromosome of said skin cells or inputting at least one value of a determined expression level of at least one gene of said skin cells;
h) comparing said value of the determined methylation level with stored data representing a correlation between the methylation level of said CpG-dinucleotide and the observed facial aging progression of at least one human individual or comparing said value of gene expression level with stored data representing a correlation between the gene expression level and the observed facial aging progression of at least one human individual; and
i) displaying the predicted facial aging progression.

17. A kit for determining the predicted facial aging progression of human skin according to the method of any one of the claims 1 to 8, comprising at least one oligonucleotide primer for amplifying and/or sequencing at least one CpG-nucleotides of at least one nucleotide sequence or at least one oligonucleotide primer for amplifying and/or sequencing fractions of at least one exon of at least one nucleotide sequence.

**Fig. 1**

**Fig. 2**

**Fig. 3**

**Fig. 4**

Fig. 5

## EUROPEAN SEARCH REPORT

Europäisches Patentamt
European Patent Office
Office européen des brevets

Application Number

**EP 23 18 1093**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/117986 A1 (LEADER MEDICAL GENETICS AND GENOMICS CO LTD [TH]) 28 June 2018 (2018-06-28) * claim 10 * | 17 | INV. C12Q1/6809 G16B20/20 |
| X | US 6 063 567 A (GALLIE BRENDA L [CA] ET AL) 16 May 2000 (2000-05-16) * claim 11 * | 17 | |
| X | RICCARDOE MARIONI ET AL: "Association of facial ageing with DNA methylation and epigenetic age predictions", CLINICAL EPIGENETICS, BIOMED CENTRAL LTD, LONDON, UK, vol. 10, no. 1, 8 November 2018 (2018-11-08), pages 1-3, XP021262404, ISSN: 1868-7075, DOI: 10.1186/S13148-018-0572-2 * page 2, left col., par. 2; Fig. 1a * | 1-13,15, 16 | |
| X | ZONARI ALESSANDRA ET AL: "Senotherapeutic peptide treatment reduces biological age and senescence burden in human skin models", NPJ AGING, vol. 9, no. 1, 22 May 2023 (2023-05-22), pages 1-15, XP093106990, ISSN: 2731-6068, DOI: 10.1038/s41514-023-00109-1 Retrieved from the Internet: URL:https://www.ncbi.nlm.nih.gov/pmc/articles/PMC10203313/pdf/41514_2023_Article_109.pdf> * abstract; page 2 left and right col. * | 14 | TECHNICAL FIELDS SEARCHED (IPC) C12Q G06F G16B |

-/--

The present search report has been drawn up for all claims

| Place of search | Date of completion of the search | Examiner |
|---|---|---|
| Munich | 12 December 2023 | Leber, Thomas |

EPO FORM 1503 03.82 (P04C01)

page 1 of 3

Europäisches
Patentamt
European
Patent Office
Office européen
des brevets

## EUROPEAN SEARCH REPORT

Application Number

EP 23 18 1093

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARIANA BORONI ET AL: "Highly accurate skin-specific methylome analysis algorithm as a platform to screen and validate therapeutics for healthy aging", CLINICAL EPIGENETICS, BIOMED CENTRAL LTD, LONDON, UK, vol. 12, no. 1, 13 July 2020 (2020-07-13), pages 1-16, XP021279253, ISSN: 1868-7075, DOI: 10.1186/S13148-020-00899-1 * the whole document * | 1-17 | |
| A | WO 2019/143845 A1 (UNIV CALIFORNIA [US]; NAT INSTITUTES OF HEALTH [US]) 25 July 2019 (2019-07-25) * claim 1; page 2, lines 4-25 * | 1-17 | |
| A | MARTIN RENAN PAULO ET AL: "Transcriptomic and histological analysis of exposed facial skin areas wrinkled or not and unexposed skin", MOLECULAR BIOLOGY REPORTS, SPRINGER NETHERLANDS, NL, vol. 49, no. 3, 1 December 2021 (2021-12-01), pages 1669-1678, XP037699182, ISSN: 0301-4851, DOI: 10.1007/S11033-021-06973-Y [retrieved on 2021-12-01] * abstract * | 1-17 | TECHNICAL FIELDS SEARCHED (IPC) |

-/--

The present search report has been drawn up for all claims

| Place of search | Date of completion of the search | Examiner |
|---|---|---|
| Munich | 12 December 2023 | Leber, Thomas |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 2 of 3

Europäisches
Patentamt

European
Patent Office

Office européen
des brevets

## EUROPEAN SEARCH REPORT

Application Number

EP 23 18 1093

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BOBROV EUGENE ET AL: "PhotoAgeClock: deep learning algorithms for development of non- invasive visual biomarkers of aging", AGING 2018, vol. 10, no. 11, 9 November 2018 (2018-11-09), pages 3249-3259, XP055912229, Retrieved from the Internet: URL:https://www.ncbi.nlm.nih.gov/pmc/artic les/PMC6286834/pdf/aging-10-101629.pdf> * the whole document * | 1-17 | |

-----

TECHNICAL FIELDS
SEARCHED       (IPC)

The present search report has been drawn up for all claims

| Place of search | Date of completion of the search | Examiner |
|---|---|---|
| Munich | 12 December 2023 | Leber, Thomas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 3 of 3

## ANNEX TO THE EUROPEAN SEARCH REPORT
## ON EUROPEAN PATENT APPLICATION NO.

**EP 23 18 1093**

This annex lists the patent family members relating to the patent documents cited in the above-mentioned European search report.
The members are as contained in the European Patent Office EDP file on
The European Patent Office is in no way liable for these particulars which are merely given for the purpose of information.

**12-12-2023**

| Patent document cited in search report | Publication date | Patent family member(s) | Publication date |
|---|---|---|---|
| WO 2018117986 A1 | 28-06-2018 | NONE | |
| US 6063567 A | 16-05-2000 | AU 3005595 A | 09-02-1996 |
| | | CA 2194696 A1 | 25-01-1996 |
| | | GB 2304411 A | 19-03-1997 |
| | | US 5550020 A | 27-08-1996 |
| | | US 6063567 A | 16-05-2000 |
| | | WO 9601908 A1 | 25-01-1996 |
| WO 2019143845 A1 | 25-07-2019 | EP 3740589 A1 | 25-11-2020 |
| | | US 2020347461 A1 | 05-11-2020 |
| | | WO 2019143845 A1 | 25-07-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HORVATH**. *Genome Biology*, 2013, vol. 14, R115
- **REINDERS et al.** Genome-wide, high-resolution DNA methylation profiling using bisulfite-mediated cytosine conversion. *Genome Res*, March 2008, vol. 18 (3), 469-76
- **VOLZKE et al.** Cohort Profile Update: The Study of Health in Pomerania (SHIP). *Int J Epidemiol*, 2022, vol. 51, e372-e383
- **ARYEE et al.** Minfi: a flexible and comprehensive Bioconductor package for the analysis of Infinium DNA methylation microarrays. *Bioinformatics*, 2014, vol. 30, 1363-9
- **RITCHIE et al.** limma powers differential expression analyses for RNA-sequencing and microarray studies. *Nucleic Acids Res*, 2015, vol. 43, e47
- **FRIEDMAN et al.** Regularization Paths for Generalized Linear Models via Coordinate Descent. *Journal of Statistical Software*, 2010, vol. 33, 1-22

- **HOLZSCHECK et al.** Multi-omics network analysis reveals distinct stages in the human aging progression in epidermal tissue. *AGING*, 2020, vol. 12
- **RADDATZ, G et al.** Aging is associated with highly defined epigenetic changes in the human epidermis. *EPIGENETICS CHROMATIN*, 2013, vol. 6 (1), 36
- **BURGER, L et al.** Identification of active regulatory regions from DNA methylation data. *NUCLEIC ACIDS RES*, 2013, vol. 41 (16), e155
- **BOLGER, A. M.** ; **LOHSE, M** ; **USADEL, B**. Trimmomatic: a flexible trimmer for Illumina sequence data. *Bioinformatics*, 2014, vol. 30, 2114-2120
- **PATRO, R.** ; **DUGGAL, G.** ; **LOVE, M. I** ; **IRIZARRY, R. A** ; **KINGSFORD, C**. Salmon provides fast and bias-aware quantification of transcript expression. *Nat. Methods*, 2017, vol. 14, 417-419